# EUROPEAN PATENT APPLICATION

(11) **EP 1 649 800 A1**
(43) Date of publication of application: **26.04.2006**
(21) Application number: 05022923.6
(22) Date of filing: 20.10.2005
(51) Int. Cl.: A61B 1/04

(54) **Electronic endoscope apparatus and image recording method**

(30) Priority: 20.10.2004 JP 2004305041
(71) Applicant: Fujinon Corporation, Kita-ku Saitama-shi Saitama-ken (JP); FUJI PHOTO FILM CO., LTD, Kanagawa-ken (JP)
(72) Inventor: Abe, Kazunori, Saitama-shi Saitama-ken (JP); Donomae, Yoshifumi c/o Fujifilm Software Co., Ltd, Kawasaki-shi Kanagawa-ken (JP)
(74) Representative: Klunker . Schmitt-Nilson . Hirsch

(57) **Abstract**

When an image obtained by an electronic endoscope apparatus is recorded, the image is recorded in a state where whether or not the image has been subjected to image processing can be judged with certainty. Recording image selectionmeans (15a, 13, 19, 26) selects either an original image without the image processing by image processing means (21, 20) or a processed image having been subjected to the image processing, as the image to be recorded by image recording means (27, 28, 29). Image type information generation means (19) generates image type information representing whether the image selected by the recording image selection means is the original image or the processed image, and the image recording means records the selected image in relation to the image type information in response to operation by an operator using operation means (15b., 13, 19).

## Description

### BACKGROUND OF THE INVENTION

### Field of the Invention

The present invention relates to an electronic endoscope apparatus. More specifically, the present invention relates to improvement in a function of recording an image obtained by an electronic endoscope apparatus.

### Description of the Related Art

An electronic endoscope apparatus generally has an image recording function for recording an image obtained by the apparatus in a recording device such as a memory or a hard disc, in order to store an observation record (see Japanese Unexamined Patent Publication No. 2002-272679, for example).

Images obtained by an electronic endoscope apparatus represent observation targets having special shapes or colors in many cases. Therefore, reflected illumination light appears too bright therein or contrast is extremely low in a considerable number of cases. Such images are not appropriate for observation in many cases if used as they are. For this reason, some electronic endoscope apparatuses have an image processing function for converting images obtained by the apparatuses into images appropriate for observation (see Japanese Unexamined Patent Publication No. 2002-272679, for example).

Actually, in diagnosis using an electronic endoscope apparatus in the field of medicine, the image processing function is turned on or off as necessary when an endoscopist examines the inside of the body of a patient, while an image important for diagnosis is recorded occasionally by use of the image recording function. The image is pasted later on an electronic medical chart or the like.

In the case of diagnoses on presence or absence of pathologic changes or degrees thereof in observation target areas by use of images obtained by an electronic endoscope apparatus, a physician often makes diagnoses based on subtle color changes or protuberances in the observation target areas. Consequently, the manner (criterion) of the diagnoses changes, depending on whether or not the images used for the diagnoses have been subjected to image processing.

Meanwhile, the image recording function of an electronic endoscope apparatus enables recording of an original image with no image processing and a processed image having been subjected to image processing.

Therefore, in the case where a physician does not know whether an image attached to an electronic medical chart is an original image or a processed image, the physician may make a wrong diagnosis by referring to the image attached to the medical chart.

However, in reality, no specific means has been available for informing a diagnostician whether or not an image recorded by an electronic endoscope apparatus has been subjected to image processing.

As such means, a physician may write in an electronic medical chart whether an image recorded by an electronic endoscope apparatus is an original image or a processed image at the time the image is attached to the medical chart. However, a physician may forget to write this information, or write erroneous information. Therefore, it is preferable for an image obtained by an electronic endoscope apparatus to be recorded in a recording device by the electronic endoscope apparatus in such a manner that distinction can be made with certainty regarding whether the image has been processed or has not been processed.

### SUMMARY OF THE INVENTION

The present invention has been conceived based on consideration of the above circumstances. An object of the present invention is therefore to provide an electronic endoscope apparatus enabling recording of an image obtained by the apparatus in such a manner that distinction can be made with certainty regarding whether the image has been processed or has not been processed.

An electronic endoscope apparatus of the present invention is an electronic endoscope apparatus comprising a scope having an imaging device for imaging an observation target, image processing means for carrying out image processing on an image obtained by the imaging device, and image recording means for recording the image obtained by the imaging device. The electronic endoscope apparatus of the present invention comprises:
recording image selection means for enabling selection between an original image without the image processing and a processed image having been subjected to the image processing, as the image to be recorded by the image recording means;
image type information generation means for generating image type information representing whether the image for recording selected by the recording image selection means is the original image or the processed image; and
operation means for enabling an operator of the electronic endoscope apparatus to cause the image recording means to record the image. In response to operation of the operationmeans, the image recording means records the image selected by the recording image selection means, related to the image type information.

In the electronic endoscope apparatus of the present invention, the recording image selection means has a first operation unit for making the selection while the operation means has a second operation unit for the operation. In this case, it is preferable for the first operation unit and the second operation unit to be installed to the scope.

In the electronic endoscope apparatus of the present invention, the image recording means may record correspondence information representing correspondence between information for identifying the image to be recorded and the image type information of the image, separately from the image. Alternatively, the image recording means may record the image type information by attaching the image type information to the image.

An image recording method of the present invention for an electronic endoscope apparatus is an image recording method for an electronic endoscope apparatus comprising a scope having an imaging device for imaging an observation target, image processing means for carrying out image processing on an image obtained by the imaging device, and image recording means for recording the image obtained by the imaging device. The image recording method of the present invention comprises the steps of:
receiving selection between an original image without the image processing and a processed image having been subjected to the image processing, as the image to be recorded by the image recording means;
generating image type information representing whether the image selected for recording is the original image or the processed image;
receiving operation by an operator of the electronic endoscope apparatus for causing the image recording means to record the image; and
causing the image recording means to record the selected image in relation to the image type information in response to the operation.

In the image recording method of the present invention, the step of causing the image recording means to record the image may be a step of causing the image recording means to record correspondence information representing correspondence between information for identifying the image to be recorded and the image type information of the image, separately from the image. Alternatively, the step of causing the image recording means to record the image may be a step of causing the image recording means to record the image type information by attaching the image type information to the image.

The image type information may include information for identifying a type of the image processing, in addition to the information on distinction between the original image and the processed image.

The image processing may include only a single type of processing or a plurality of types of processing.

According to the electronic endoscope apparatus of the present invention, the recording image selection means enables the selection between the original image without the image processing by the image processing means and the processed image having been subjected to the image processing, as the image to be recorded by the recording means. The image type information generation means then generates the image type information representing whether the image selected by the recording image selection means to be recorded is the original image or the processed image, and the image recording means records the image selected by the recording image selection means, related to the image type information in response to the operation by the operator using the operation means. Therefore, the image can be recorded by being related to the information representing whether the image is the original image or the processed image, in response to the operation for recording the image. In this manner, the image can be recorded in a state where distinction can surely be made regarding whether the image has been processed or not.

### BRIEF DESCRIPTION OF THE DRAWINGS

Figure 1 is a block diagram showing the configuration of an electronic endoscope apparatus of an embodiment of the present invention; and
Figure 2 shows an example of images displayed on a monitor.

### DESCRIPTION OF THE PREFERRED EMBODIMENT

Hereinafter, an embodiment of the present invention will be described.

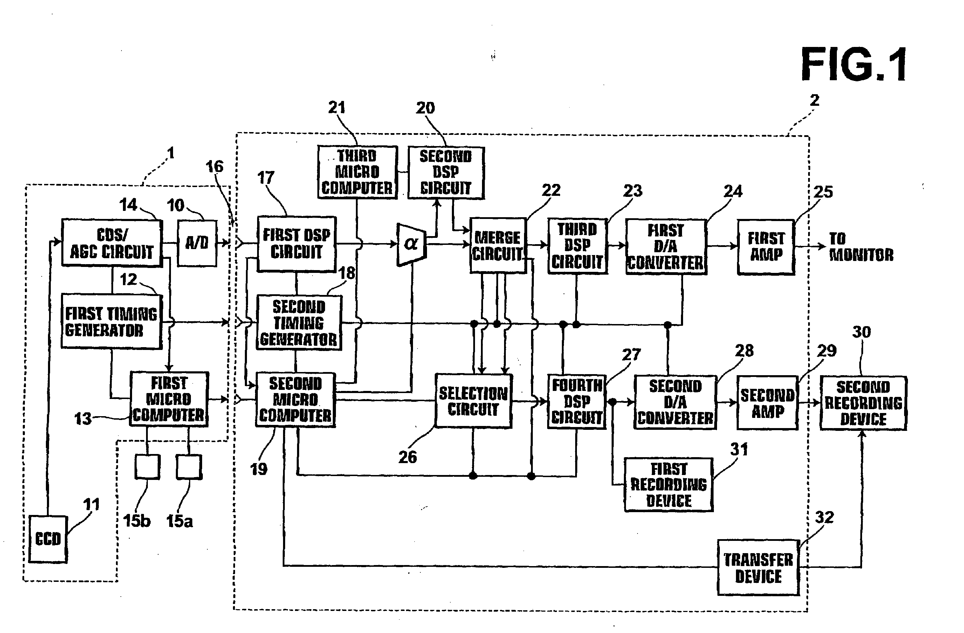

Figure 1 is a block diagram showing the configuration of an electronic endoscope apparatus of the embodiment of the present invention. The electronic endoscope apparatus comprises a scope 1 for mainly imaging an observation target and a processor 2 that is connected to the scope 1 and mainly generates signals for monitor display and for recording in a recording device by processing a signal obtained by the scope 1.

The scope 1 comprises a CCD (an imaging device) 11 installed at a tip thereof, a first timing generator 12 for generating and outputting various kinds of timing signals for the scope 1 including a driving signal for the CCD 11, a first microcomputer 13 for controlling various circuits in the scope 1, a correlated double sampling/automatic gain control (CDS/AGC) circuit 14 for carrying out correlated double sampling and automatic gain control on an image signal as an analog electric signal output from the CCD 11, an A/D converter 10, a first button switch (a first operation unit) 15a and a second button switch (a second operation unit) 15b for carrying out predetermined operations. The first button switch 15a and the second button switch 15b are connected to the first microcomputer 13 so that information on operation of the switches can be sent to the first microcomputer 13.

The processor 2 comprises a first DSP (Digital Signal Processor) circuit 17, a second timing generator 18, a second microcomputer 19, a second DSP circuit 20, a third microcomputer 21, a merge circuit 22, a third DSP circuit 23, a first D/A converter 24, a first amplifier 25, a selection circuit 26, a fourth DSP circuit 27, a second D/A converter 28, and a second amplifier 29. The first DSP circuit 17 generates a video signal such as a Y (luminance) signal and a C (color difference) signal, based on the signal sent from the CDS/AGC circuit 14. The first DSP circuit 17 also carries out various kinds of processing on the video signal for generating a color image. The second timing generator 18 synchronizes with the first timing generator 12 for generating and outputting various kinds of timing signals for the processor 2 including a driving signal for the first DSP circuit 17. The second microcomputer 19 receives information on the scope 1 from the first microcomputer 13, and controls the circuits in the processor 2 based on the information. The second DSP circuit 20 carries out predetermined image processing on the signal obtained by the first DSP circuit 17. The third microcomputer 21 controls the second DSP circuit 20. The merge circuit 22 merges and stores the original signal obtained by the first DSP circuit 17 with a processed signal having been subjected to the predetermined image processing by the second DSP circuit 20. The third DSP circuit 23 generates R (Red), G (Green), and B (Blue) signals for an RGB monitor, based on the Y signal and the C signal read from the merge circuit 22. The first D/A converter 24 converts the RGB signals into an analog signal for the monitor. The first amplifier 25 amplifies the analog signal for the monitor. The selection circuit 26 selects either the original signal obtained by the first DSP circuit 17 or the processed signal having been subjected to the predetermined image processing by the second DSP circuit 20 from the merge circuit 22, and outputs the selected signal. The fourth DSP circuit 27 generates RGB signals for recording, based on the Y signal and the C signal as the original signal or the processed signal output from the selection circuit 26. The second D/A converter converts the RGB signals into an analog signal for recording. The second amplifier 29 amplifies the analog signal for recording. The second amplifier 29 is connected to a recording device 30 such as a memory, a hard disc, or a printer, and the image represented by the analog signal output from the second amplifier 29 is recorded in the recording device 30.

A switch α is located between the first DSP circuit 17 and the merge circuit 22 for changing a path of the signal. By controlling the switch, selection can be made on whether the original signal obtained by the first DSP circuit 17 is sent to the merge circuit 22 as it is, or as the processed signal generated via the second DSP circuit 20 through the predetermined image processing thereon, or both as the original signal and as the processed signal.

The scope 1 and the processor 2 are connected to each other by a connector 16 so that the signal obtained by the CCD 11 and various control signals are conveyed between the scope 1 and the processor 2.

Each of the circuits is powered by a power supply circuit that is not shown, and the power supply circuit is connected to a commercial power source.

In the above configuration, the third microcomputer 21 and the second DSP circuit 20 act as the image processing means of the present invention while the fourth DSP circuit 27, the second D/A converter 28, and the second amplifier 29 act as the image recording means. The first button switch 15a, the first microcomputer 13, the second microcomputer 19, and the selection circuit 26 act as the recording image selection means of the present invention while the second microcomputer 19 acts as the image type information generation means. The second button switch 15b, the first microcomputer 13, and the second microcomputer 19 function as the operation means of the present invention.

As the predetermined image processing described above can be used hypertone processing (proposed by Fuji Photo Film Co., Ltd.) or the like. The hypertone processing adopts compression processing of an expressible density range, as has been proposed in Japanese Unexamined Patent Publication No . 9(1997)-018704, for example. In the hypertone processing, a blurry image is generated from the signal obtained by the CCD 11, for representing a low-frequency component in the original image represented by the signal. A non-linear conversion image is then obtained by converting a part of the blurry image whose luminance exceeds a predetermined luminance value into a part of zero luminance while converting a part whose luminance is the predetermined luminance value or lower into a part having a luminance value corresponding to the luminance value of the blurry image. The non-linear conversion image is added to the original image for obtaining an image wherein a dark area has been converted into an area of higher luminance.

Operation of the electronic endoscope apparatus will be described next.

When the electronic endoscope apparatus is turned on, power is supplied from the power supply circuit to the circuits therein. The driving signal output from the first timing generator 12 drives the CCD 11, and the CCD 11 images the observation target. The image signal is sent to the CDS/AGC circuit 14. The CDS/AGC circuit 14 carries out correlated double sampling on the signal while amplifying the signal with a predetermined gain, and the signal is supplied to the first DSP circuit 17 as the video signal.

The video signal output from the CDS/AGC circuit 14 is converted into a digital signal by the A/D converter 10, and subjected to predetermined processing by the first DSP circuit 17. The original signal is sent to the merge circuit 22 as it is, or as the processed signal via the second DSP circuit 20, or as the original signal and the processed signal. The second microcomputer 19 selects in which of the three forms the signal obtained by the first DSP circuit 17 is sent to the merge circuit 22, by controlling the switch α according to a setting made by a user. In this embodiment, the switch α has been set to send both the original signal and the processed signal to the merge circuit 22. Therefore, the merge circuit 22 stores both the original signal and the processed signal.

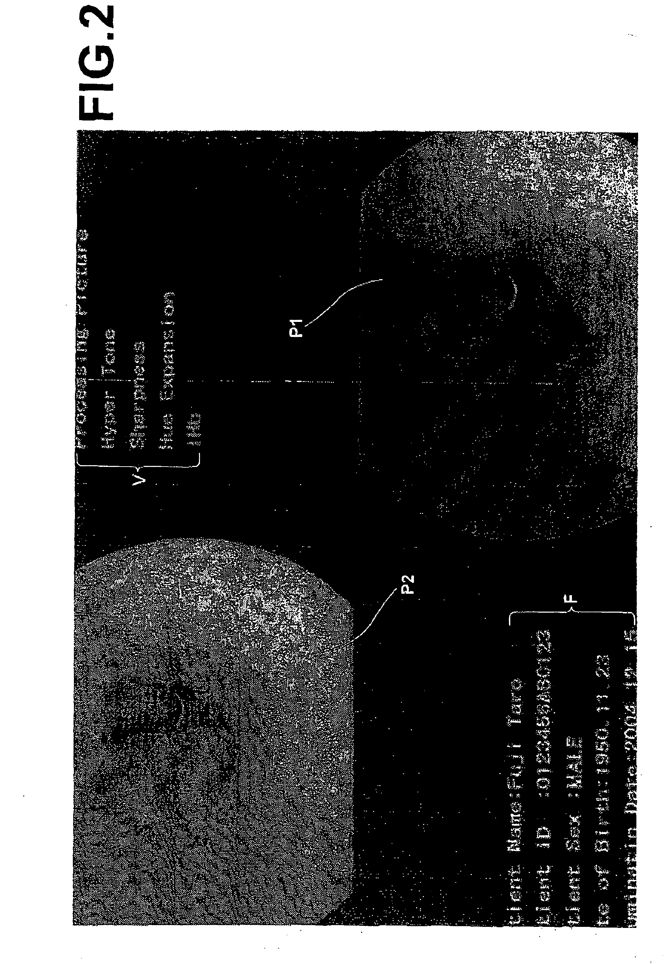

In response to control by the second microcomputer 19, the third DSP circuit 23 receives the original signal, or the processed signal, or both, from the merge circuit 22. The third DSP circuit 23 carries out various kinds of processing on the received signal, and the signal is converted into a signal for displaying the image represented by the original signal, or the image represented by the processed signal, or both on a monitor. The signal is sent to the D/A converter 24 and the first amplifier 25, and output as a video signal for the monitor. Based on the video signal, the image of the observation target is displayed on the monitor as shown in Figure 2. In Figure 2, both of the images are shown on the monitor. An image P1 represented by the original signal, an image P2 represented by the processed signal, and character information including patient information F and a type v of the image processing are displayed together on the monitor.

Selection of the type of the image to be recorded in the recording device 30 (either the original image or the processed image) and recording timing control can be made by operation of the first button switch 15a and the second button switch 15b on the scope 1. For example, the first button switch 15a has a function of alternately changing the type of the image to be recorded between the original image and the processed image at each time the button switch 15a is pressed. The second button switch 15b has a function of receiving an image recording instruction for freezing the image displayed on the monitor when pressed once, and for recording the image (either the original image or the processed image) corresponding to the selected type when pressed once more.

Information on the operation of these buttons is sent to the first microcomputer 13 which sends the information to the second microcomputer 19. The second microcomputer 19 controls the selection circuit 26, the fourth DSP circuit 27, and the like according to the information, for selection of the image type and for recording of the image. The selection circuit 26 obtains either the original signal or the processed signal from the merge circuit 22 at predetermined timing, in response to the control by the second microcomputer 19 according to the operation by the user. The signal is then sent to the fourth DSP circuit 27 where various kinds of processing is carried out on the signal for converting the signal into the signal for recording the image represented by the signal. The signal is sent to a first recording device 31 wherein the signal is recorded as the digital signal. The signal is also sent to the recording device 30 as a second recording device via the second D/A converter 28 and the second amplifier 29, for recording the image.

When the image is recorded in the first recording device 31, the second microcomputer 19 recognizes the type of the image (either the original image or the processed image), and generates the image type information representing the type. The second microcomputer 19 causes the image to be recorded together with the image type information attached thereto. For example, in the case where a format of image data has tag information, the image type information may be written in the tag information. The second microcomputer 19 sends the same content as the information recorded in the first recording device 31, to the second recording device 30 via communication means including transfer means 32. The second recording device 30 receives the image type information representing the type of the image (either the original image or the processed image), and attaches the image type information to the image to be recorded therein. In the case of the format using the tag information, the image type information may be written in the tag information.

As has been described above, according to the electronic endoscope apparatus in this embodiment, the recording image selection means comprising the first button switch 15a, the first microcomputer 13, the second microcomputer 19, and the selection circuit 26 can select either the original image without the image processing by the image processing means comprising the third microcomputer 21 and the second DSP circuit 20 or the processed image having been subjected to the image processing, as the image to be recorded by the image recording means comprising the fourth DSP circuit 27, the second D/A converter 28, and the second amplifier 29. The second microcomputer 19 as the image type information generation means generates the image type information representing whether the image for recording selected by the recording image selection means is either the original image or the processed image, and the image recording means records the image selected by the recording image selection means by relating the image to the image type information, in response to the operation by the user using the operation means comprising the second switch button 15b, the first microcomputer 13, and the second microcomputer 19. Therefore, in response to the operation for recording the image, the information representing whether or not the image has been processed can be recorded in relation to the image. In this manner, when the image obtained by the electronic endoscope apparatus is recorded, the image can be recorded in a state where whether or not the image has been processed can be judged with certainty.

In the case where the original image and the processed image are displayed together on the monitor, both of the images may be displayed as live images or either one of the images may be a still image. This manner of display can be realized by setting the third DSP circuit 23 and the second microcomputer 19 that controls the third DSP circuit 23.

The CCD 11 may be a primary-color CCD or a complementary-color CCD.

## Claims

1. An electronic endoscope apparatus comprising a scope (1) having an imaging device (11) for imaging an observation target, image processing means (21, 20) for carrying out image processing on an image obtained by the imaging device (11), and image recording means (27, 28, 29) for recording the image obtained by the imaging device (11), the electronic endoscope apparatus comprising:
recording image selection means (15a, 13, 19, 26) for enabling selection between an original image without the image processing and a processed image having been subjected to the image processing, as the image to be recorded by the image recording means (27, 28, 29);
image type information generation means (19) for generating image type information representing whether the image selected by the recording image selection means (15a, 13, 19, 26) to be recorded is the original image or the processed image; and
operation means (15b, 13, 19) for enabling an operator of the electronic endoscope apparatus to cause the image recording means (27, 28, 29) to record the image, wherein
the image recording means (19, 31) records the image selected by the recording image selection means (15a, 13, 19) while relating the image to the image type information, in response to operation of the operation means (15b, 13, 19).

2. The electronic endoscope apparatus according to Claim 1,
the recording image selection means (15a, 13, 19, 26) having a first operation unit (15a) for making the selection,
the operation means (15b, 13, 19) having a second operation unit (15b) for the operation, and
the first operation unit (15a) and the second operation unit (15b) being installed to the scope (1).

3. The electronic endoscope apparatus according to either one of Claim 1 and Claim 2, wherein the image recording means (27, 28, 29) records the image while attaching the image type information to the image.

4. The electronic endoscope apparatus according to Claim 1, wherein the image recording means (27, 28, 29) records correspondence information representing correspondence between information for identifying the image to be recorded and the image type information of the image, separately from the image.

5. The electronic endoscope apparatus according to Claim 1,
wherein the image type information includes information for identifying a type of the image processing, in addition to distinction between the original image and the processed image.

6. The electronic endoscope apparatus according to Claim 1, wherein the image processing includes a plurality of types of processing.

7. An image recording method for an electronic endoscope apparatus comprising a scope (1) having an imaging device (11) for imaging an observation target, image processing means (21, 20) for carrying out image processing on an image obtained by the imaging device (11), and image recording means (27, 28, 29) for recording the image obtained by the imaging device (11), the image recording method comprising the steps of:
receiving selection between an original image without the image processing and a processed image having been subjected to the image processing, as the image to be recorded by the image recording means (27, 28, 29);
generating image type information representing whether the image selected for recording is the original image or the processed image;
receiving operation by an operator of the electronic endoscope apparatus for causing the image recording means (27, 28, 29) to record the image; and
causing the image recording means (27, 28, 29) to record the selected image while relating the image to the image type information in response to the operation.

8. The image recording method for the electronic endoscope apparatus according to Claim 7, wherein the step of causing the image recording means (27, 28, 29) to record the image is the step of causing the image recording means (27, 28, 29) to record the image while attaching the image type information to the image.

9. The image recording method for the electronic endoscope apparatus according to Claim 7, wherein the step of causing the image recording means (27, 28, 29) to record the image is the step of causing the image recording means (27, 28, 29) to record correspondence information representing correspondence between information for identifying the image to be recorded and the image type information of the image, separately from the image.

10. The image recording method for the electronic endoscope apparatus according to Claim 7, wherein the image type information includes information for identifying a type of the image processing, in addition to distinction between the original image and the processed image.

11. The image recording method for the electronic endoscope apparatus according to Claim 7, wherein the image processing includes a plurality of types of processing.
